# EUROPEAN PATENT SPECIFICATION

(11) **EP 3 192 448 B1**
(45) Date of publication and mention of the grant of the patent: **23.08.2023**
(21) Application number: 15839396.7
(22) Date of filing: 18.06.2015
(51) Int. Cl.: A61B 8/08, A61B 8/00

(54) **ELASTICITY DETECTION PROBE**
ELASTIZITÄTSDETEKTIONSSONDE
SONDE DE DÉTECTION D'ÉLASTICITÉ

(30) Priority: 12.09.2014 CN 201420526844 U
(43) Date of publication of application: 19.07.2017
(73) Proprietor: Wuxi Hisky Medical Technologies Co., Ltd., Taihu International Science & Technology Park Wuxi Jiangsu 214000 (CN)
(72) Inventor: SHAO, Jinhua, Wuxi Jiangsu 214000 (CN); SUN, Jin, Wuxi Jiangsu 214000 (CN); DUAN, Houli, Wuxi Jiangsu 214000 (CN)
(74) Representative: Grey, Ian Michael
(86) International application number: PCT/CN2015/081866
(87) International publication number: WO 2016/037509

(56) References cited:
- CN-A- 1 674 827
- CN-A- 1 728 968
- CN-A- 101 677 807
- CN-A- 102 308 207
- CN-U- 204 274 643
- KR-A- 20090 101 678
- US-A1- 2005 203 398
- US-A1- 2006 058 649
- US-A1- 2009 306 515
- US-A1- 2011 301 468
- US-A1- 2012 078 111
- US-B1- 6 494 840
- US-B1- 6 494 840

## Description

### TECHNICAL FIELD

The present invention relates to the field of medical instruments, and more particular to an elasticity detecting probe.

### BACKGROUND

Elasticity measurement is of great significance in medical field. For example, the state of a liver can be monitored and evaluated via non-invasive detection of elasticity of the liver, thereby allowing a timely and effective treatment. In the prior art, the switch for controlling on/off of a probe is provided on the probe. In the conventional method, when an elasticity measurement is performed, the operator holds the probe in his/her hand, moves the probe to the detection area, and then turns on the probe to conduct the detection. This setting manner that the switch is set on the probe causes changes in the angle and position of the probe when the switch is being switched on, so that the position and angle measured by the probe will not be the originally selected ones, and the positions and angles in each measurement are different. Thus, the elastic modulus calculated using an algorithm will deviate from the true value, hereby affecting the stability and accuracy of the measurement result. At the same time, since the start switch is mounted on the probe, the operator not only needs to hold the probe, but also needs to repeatedly press the start switch with his/her hand, thereby increasing the difficulty of operation. The inconvenience in operation will prolong the measurement duration, increase the patient's waiting time, and affect the doctor's efficiency. The existence of the start switch will also affect the manner of holding the probe to some extent, easily making the operator to form a wrong operating habit.

Document US6494840B1 describes a portable ultrasound palpation device for measuring the Young's modulus and the thickness of a soft tissue layer includes a hand-holdable palpation probe having an ultrasonic transceiver connected in series with a load cell.

### SUMMARY

The present invention provides an elasticity detecting probe, for solving the problem of the prior art that when a switch is turned on, the position of detecting probe will move.

The technical solution of the present invention is realized as follows:
The present invention provides an elasticity detecting probe including a probe body and a control switch for controlling on/off of the probe body, where the probe body and the control switch are separately arranged, and the probe body and the control switch are coupled to each other electrically or wirelessly, where the elasticity detecting probe further includes: a control system and a display device, where the control system is configured to collect motion state parameter of the probe body and display the motion state parameters on the display device, where the motion state parameters are reflected on the display device connected to the control system such that the operator can understand the motion state of the probe body by observing the parameters displayed on the display device.

Further, the control switch is a mechanical switch, preferably a pedal switch.

Further, the control switch is a sensor, preferably a voice-operated switch or a light-operated switch.

Further, when the control switch is the voice-operated switch, the control system includes a memory and a processor, where the memory stores a plurality of pre-recorded voice clips, each of which corresponds to a control command. The voice-operated switch is configured to collect a voice clip, and the processor is configured to compare the voice clip collected by the voice-operated switch with the voice clips stored in the memory and send a corresponding control command to the probe body when the voice clip collected by the voice-operated switch is matched with a voice clip stored in the memory.

### BRIEF DESCRIPTION OF THE DRAWINGS

To describe technical solutions in embodiments of the present invention or the prior art more clearly, the following briefly describes the accompanying drawings required in the description of embodiments or the prior art, apparently, the accompanying drawings illustrate only some exemplary embodiments of the present invention, and those skilled in the art can derive other drawings from these drawings without creative efforts.
FIG. 1 is a schematic structural diagram of an elasticity detecting probe according to an embodiment of the present invention;
FIG. 2 is a schematic structural diagram of another elasticity detecting probe according to an embodiment of the present invention.

In the drawings: 1. probe body; 2. control system; 21. memory; 22. processor; 3. control switch; 4. display device.

### DESCRIPTION OF EMBODIMENTS

The technical solutions in the embodiments of the present invention will be described clearly and comprehensively with reference to the accompanying drawings in embodiments of the present invention, apparently, the embodiments described are only some exemplary embodiments of the present invention, not all embodiments. Other embodiments derived by those skilled in the art on the basis of the embodiments herein without any creative effort fall within the protection scope of the present invention.

An elasticity detecting probe of the present embodiment includes a probe body and a control switch, wherein the probe body and the control switch are separately arranged, and the probe body and the control switch are coupled to each other electrically or wirelessly. That is, the probe body is not provided with a control switch. In the elasticity detecting probe of the present embodiment, the probe body and the control switch are separately arranged, so that an operator can operate the control switch to turn on/off the probe body by using other body parts rather than the two hands, thereby preventing the offset of the probe body when the control switch is turned on, and improving the detection accuracy.

The control switch in the present embodiment may be a mechanical switch or an inductive switch. When a mechanical switch is adopted, the mechanical switch may preferably be set as a pedal-type. The choice of switch design in the pedal-type is in consideration of that the two hands operating the probe body are incapable of operating on other devices during the elastic measurement, so the control switch has to be operated by other body parts; meanwhile, taking into account the accuracy and convenience, as well as the sitting posture of the operator during the detection, the switch is finally set at the foot of the operator, being formed to be a pedal switch. It is convenient for the operator to operate on the switch by using a pedal switch. No matter with a touching start switch or a long-pressing start switch, the operator can quickly and accurately complete the control switch operation by using his/her feet, opening or closing the probe body.

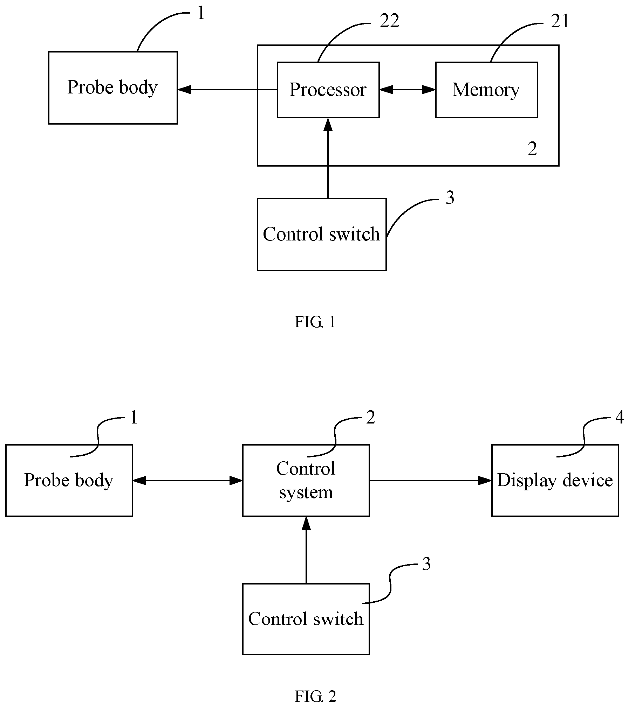

The mechanical switch described above may also be replaced with the inductive switch, for example, a sensor may be used as the control switch. As shown in FIG. 1, in a preferred embodiment adopting the inductive switch, the voice-operated switch can be used as the control switch 3 to control on/off of the probe body 1. When the voice-operated switch is adopted, several voice clips of the operator can be pre-recorded and stored in the memory 21 of a control system 2, and the sound wave information corresponding to the voice clips are generated by a processor 22 of the control system 2. There is a one-to-one correspondence between the voice clip and the sound wave information. Each piece of sound wave information corresponds to a function instruction, and multiple pieces of sound wave information collectively form an instruction set for operating the probe body 1. When the operator operates the probe body 1, it is only need to send a voice clip corresponding to the sound wave information matching with the operation command. The sound sensor, i.e. the control switch 3, collects the voice clip from the operator and transmits the voice clip to the processor 22 of the control system 2. The processor 22 then compares the collected voice clip with the voice clips stored in the memory 21. Following the comparison, if the voice clip transmitted by the operator is matched with a recorded voice clip, then the processor 22 transmits a corresponding control command to the probe body 1 for activating a function corresponding to the sound wave information converted by the voice clip, therefore one-time voice control is completed. The above-mentioned voice-operated switch can also be replaced by a light-operated switch, which has the same working principle as the voice-operated switch. It should be clearly noted that, the control system 2 in FIG. 1 is illustrated as other parts separated from the probe body 1 and the control switch 3, it will be understood by those skilled in the art that the control system 2 may also be a part of the probe body 1 or be a part of the control switch 3, the changes thereof are within the protection scope of the present invention. The inductive switch used as the control switch 3 not only facilitates the operator to focus on the controlling of the probe body 1, but also facilitates the operator's operation of the control switch 3, thereby greatly improving the working efficiency of the operator.

In addition, as shown in FIG. 2, in order to facilitate the operator to have a more intuitive understanding of the motion state of the probe body 1, the control system 2 can collect the motion state parameters of the probe body 1 in real time via, for example, a sensor, a camera or the like. And these parameters are reflected on the display device 4 connected to the control system 2, and the operator can clearly understand the motion state of the probe body 1 by observing the parameters displayed on the display device 4. Similarly, although the probe body 1, the control system 2, the control switch 3 and the display device 4 are illustrated as separate components in FIG. 2, it is possible to integrate two or more of these components together, as long as making sure that the probe body 1 and the control switch 3 are separated from each other.

The above description is only for the purpose of describing preferred embodiments of the present invention and is not to be construed as limiting the present invention. Any modifications, equivalent substitutions, improvements, etc., within the spirit and principle of the present invention should be within the protection scope of the present invention.

## Claims

1. An elasticity detecting probe, comprising: a probe body (1) and a control switch (3) for controlling switching on/off of probing by the probe body (1), wherein the probe body (1) and the control switch (3) are separately arranged, and the probe body (1) and the control switch (3) are coupled to each other electrically or wirelessly, the probe further comprising: a control system (2) and a display device (4), and **characterized in that** the control system (2) is configured to collect motion state parameter of the probe body (1) and display the motion state parameters on the display device (4), wherein the motion state parameters are reflected on the display device (4) connected to the control system (2) such that the operator can understand the motion state of the probe body (1) by observing the parameters displayed on the display device (4).

2. The elasticity detecting probe according to claim 1, wherein the control switch (3) is a mechanical switch.

3. The elasticity detecting probe according to claim 2, wherein the mechanical switch is a pedal switch.

4. The elasticity detecting probe according to claim 1, wherein the control switch (3) is a sensor.

5. The elasticity detecting probe according to claim 4, wherein the control switch (3) is a light-operated switch.

6. The elasticity detecting probe according to claim 4, wherein the control switch (3) is a voice-operated switch.

7. The elasticity detecting probe according to claim 6, further comprising: a control system (2); wherein the control system (2) comprises a memory (21) and a processor (22); the memory (21) stores a plurality of pre-recorded voice clips; each voice clip corresponds to a control command; the voice-operated switch is configured to collect a voice clip; the processor (22) is configured to compare the voice clip collected by the voice-operated switch with the voice clips stored in the memory (21), and send a corresponding control command to the probe body (1) when the voice clip collected by the voice-operated switch is matched with a voice clip stored in the memory.

## Patentansprüche

1. Elastizitätsdetektionssonde, umfassend: einen Sondenkörper (1) und einen Steuerschalter (3) zum Steuern des Ein- und Ausschaltens der Sondierung durch den Sondenkörper (1), wobei der Sondenkörper (1) und der Steuerschalter (3) getrennt angeordnet sind und der Sondenkörper (1) und der Steuerschalter (3) elektrisch oder drahtlos aneinandergekoppelt sind, wobei die Sonde ferner Folgendes umfasst: ein Steuersystem (2) und eine Anzeigevorrichtung (4), und **dadurch gekennzeichnet, dass** das Steuersystem (2) dazu konfiguriert ist, Bewegungszustandsparameter des Sondenkörpers (1) zu erfassen und die Bewegungszustandsparameter auf der Anzeigevorrichtung (4) anzuzeigen, wobei die Bewegungszustandsparameter auf der Anzeigevorrichtung (4), die mit dem Steuersystem (2) verbunden ist, reflektiert werden, sodass der Bediener den Bewegungszustand des Sondenkörpers (1) durch Beobachten der auf der Anzeigevorrichtung (4) angezeigten Parameter verstehen kann.

2. Elastizitätsdetektionssonde nach Anspruch 1, wobei der Steuerschalter (3) ein mechanischer Schalter ist.

3. Elastizitätsdetektionssonde nach Anspruch 2, wobei der mechanische Schalter ein Pedalschalter ist.

4. Elastizitätsdetektionssonde nach Anspruch 1, wobei der Steuerschalter (3) ein Sensor ist.

5. Elastizitätsdetektionssonde nach Anspruch 4, wobei der Steuerschalter (3) ein lichtgesteuerter Schalter ist.

6. Elastizitätsdetektionssonde nach Anspruch 4, wobei der Steuerschalter (3) ein sprachgesteuerter Schalter ist.

7. Elastizitätsdetektionssonde nach Anspruch 6, ferner umfassend: ein Steuersystem (2); wobei das Steuersystem (2) einen Speicher (21) und einen Prozessor (22) umfasst; der Speicher (21) eine Vielzahl von vorab aufgezeichneten Sprachclips speichert; jeder Sprachclip einem Steuerbefehl entspricht; der sprachgesteuerte Schalter dazu konfiguriert ist, einen Sprachclip zu erfassen; der Prozessor (22) dazu konfiguriert ist, den durch den sprachgesteuerten Schalter erfassten Sprachclip mit den im Speicher (21) gespeicherten Sprachclips zu vergleichen und einen entsprechenden Steuerbefehl an den Sondenkörper (1) zu senden, wenn der durch den sprachgesteuerten Schalter erfasste Sprachclip mit einem im Speicher gespeicherten Sprachclip übereinstimmt.

## Revendications

1. Sonde de détection d'élasticité, comprenant : un corps de sonde (1) et un interrupteur de commande (3) pour commander l'activation/la désactivation du sondage par le corps de sonde (1), dans laquelle le corps de sonde (1) et l'interrupteur de commande (3) sont agencés séparément, et le corps de sonde (1) et l'interrupteur de commande (3) sont couplés l'un à l'autre électriquement ou sans fil, la sonde comprenant en outre : un système de commande (2) et un dispositif d'affichage (4), et **caractérisée en ce que** le système de commande (2) est configuré pour collecter un paramètre d'état de mouvement du corps de sonde (1) et afficher les paramètres d'état de mouvement sur le dispositif d'affichage (4), dans laquelle les paramètres d'état de mouvement sont reflétés sur le dispositif d'affichage (4) relié au système de commande (2) de sorte que l'opérateur puisse comprendre l'état de mouvement du corps de sonde (1) en observant les paramètres affichés sur le dispositif d'affichage (4).

2. Sonde de détection d'élasticité selon la revendication 1, dans laquelle l'interrupteur de commande (3) est un interrupteur mécanique.

3. Sonde de détection d'élasticité selon la revendication 2, dans laquelle l'interrupteur mécanique est un interrupteur à pédale.

4. Sonde de détection d'élasticité selon la revendication 1, dans laquelle l'interrupteur de commande (3) est un capteur.

5. Sonde de détection d'élasticité selon la revendication 4, dans laquelle l'interrupteur de commande (3) est un interrupteur actionné par la lumière.

6. Sonde de détection d'élasticité selon la revendication 4, dans laquelle l'interrupteur de commande (3) est un interrupteur à commande vocale.

7. Sonde de détection d'élasticité selon la revendication 6, comprenant en outre : un système de commande (2) ; dans lequel le système de commande (2) comprend une mémoire (21) et un processeur (22) ; la mémoire (21) stocke une pluralité de clips vocaux préenregistrés ; chaque clip vocal correspond à une instruction de commande ; l'interrupteur à commande vocale est configuré pour collecter un clip vocal ; le processeur (22) est configuré pour comparer le clip vocal collecté par l'interrupteur à commande vocale aux clips vocaux stockés dans la mémoire (21), et envoyer une instruction de commande correspondante au corps de sonde (1) lorsque le clip vocal collecté par l'interrupteur à commande vocale est mis en correspondance avec un clip vocal stocké dans la mémoire.
